# EUROPEAN PATENT APPLICATION

(11) **EP 0 838 452 A1**
(43) Date of publication of application: **29.04.1998**
(21) Application number: 96920007.0
(22) Date of filing: 30.05.1996
(51) Int. Cl.: C07C 251/48, C07C 249/04, C07C 249/12, A01N 37/50

(54) **PROCESS FOR PRODUCING N-METHYL-METHOXYIMINOACETAMIDE DERIVATIVES AND INTERMEDIATES THEREOF**

(30) Priority: 31.05.1995 JP 133646/95; 14.12.1995 JP 325696/95
(71) Applicant: MITSUBISHI CHEMICAL CORPORATION, Chiyoda-ku, Tokyo 100 (JP)
(72) Inventor: KATSURADA, Manabu Mitsubishi Chemical Corporation, Aoba-ku, Yokohama-shi Kanagawa 227 (JP); ODA, Masatsugu Mitsubishi Chemical Corporation, Aoba-ku, Yokohama-shi Kanagawa 227 (JP)
(74) Representative: VOSSIUS & PARTNER
(86) International application number: JP9601464
(87) International publication number: WO9638408

(57) **Abstract**

A method for producing an N-methyl-methoxyiminoacetamide derivative represented by the following general formula (I) 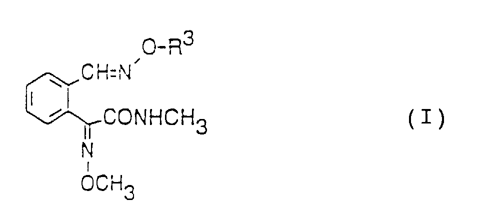 (wherein R³ represents a hydrogen atom or a group represented by a general formula -C(R⁴)(R⁵)-Ar (wherein R⁴ and R⁵ independently represent a hydrogen atom or a C₁-C₄ alkyl group and Ar is an optionally substituted aryl group or an optionally substituted heteroaryl group)), which comprises allowing an acetal derivative represented by the following general formula (III) 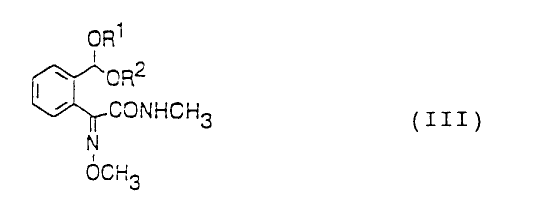 (wherein R¹ and R² independently represent a C₁-C₄ alkyl group, or R¹ and R² in combination represent a C₂-C₄ alkylene group) to react with an oxyamine derivative represented by the following general formula (II)

H₂NO-R³ (II).

The compound of general formula (I) is an excellent agricultural horticultural fungicide described in an unexamined published Japanese patent application (*kokai*) No. 7-076564.

## Description

### TECHNICAL FIELD

This invention relates to a process for the production of a methoxyiminoacetamide derivative which is useful as an agricultural horticultural fungicide and to an intermediate for producing the same.

### BACKGROUND ART

An unexamined published Japanese patent application (*kokai*) No. 7-076564 discloses an N-methyl-methoxyiminoacetamide derivative having excellent fungicidal activity. As the production method, this published application describes a method in which a methoxyiminoacetic acid ester derivative is subjected to amidation, but this method involves side reactions in some cases and therefore cannot always provide the product of interest stably.

Also, WO 94/22812 describes a method in which an N-methyl-methoxyiminoacetamide derivative is produced using 1-hydroxy-4-methoxyimino-2-methyl-3-oxo-1,2,3,4-tetrahydroisoquinoline as a starting material. However, according to the examples of this published application, the yield is merely 38% which is not satisfactory from the industrial point of view.

In addition, unexamined Japanese patent applications (*kokai*) No. 63-23852, No. 3-246268, No. 4-89464, No. 4-182461, No. 5-43533, No. 5-97768, No. 5-331124, No. 6-25132 and the like also describe methoxyiminoacetic acid derivatives having biological activities and production methods therefor. However, they have problems in that their yields are low or they cannot be applied to the N-methyl-methoxyiminoacetamide derivative described in an unexamined published Japanese patent application (*kokai*) No. 7-076564.

In consequence, great demands has been directed toward the development of a method by which a methoxyiminoacetamide derivative which is useful as an agricultural horticultural fungicide can be produced efficiently with a low cost.

The inventors of the present invention have conducted extensive studies and succeeded in obtaining the object product by the use of a novel acetal derivative as the starting material, thus resulting in the accomplishment of this invention.

### DISCLOSURE OF THE INVENTION

Accordingly, the gist of the present invention resides in a method for the production of an N-methyl-methoxyiminoacetamide derivative represented by the following general formula (I) 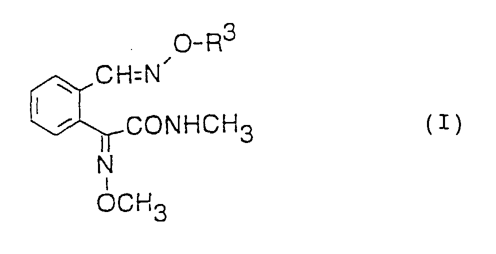 (wherein R³ represents a hydrogen atom or a group represented by a general formula -C(R⁴)(R⁵)-Ar (wherein each of R⁴ and R⁵ represents a hydrogen atom or a C₁-C₄ alkyl group, and Ar represents an optionally substituted aryl group or an optionally substituted heteroaryl group)), which comprises allowing an acetal derivative represented by the following general formula (III) 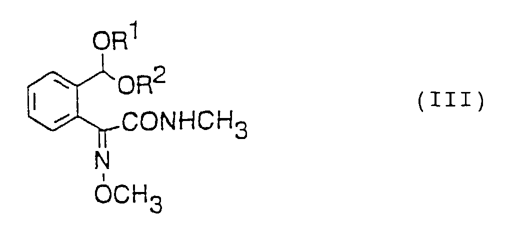 (wherein R¹ and R² independently represent a C₁-C₄ alkyl group, or R¹ and R² in combination represent a C₁-C₄ alkylene group) to react with an oxyamine derivative represented by the following general formula (II)

H₂NO-R³ (II)

(wherein R³ is as defined above). The present invention also relates to an intermediate for the production, i.e., the acetal derivative of the above general formula (III).

The present invention also relates to a series of reactions as shown in the following reaction scheme-1 starting from a 2-(2-alkoxyphenyl)-2-methoxyiminoacetic acid ester derivative (VI) is used as a starting material to the N-methyl-methoxyiminoacetamide derivative of the general formula (I), and to novel intermediates of the reaction. 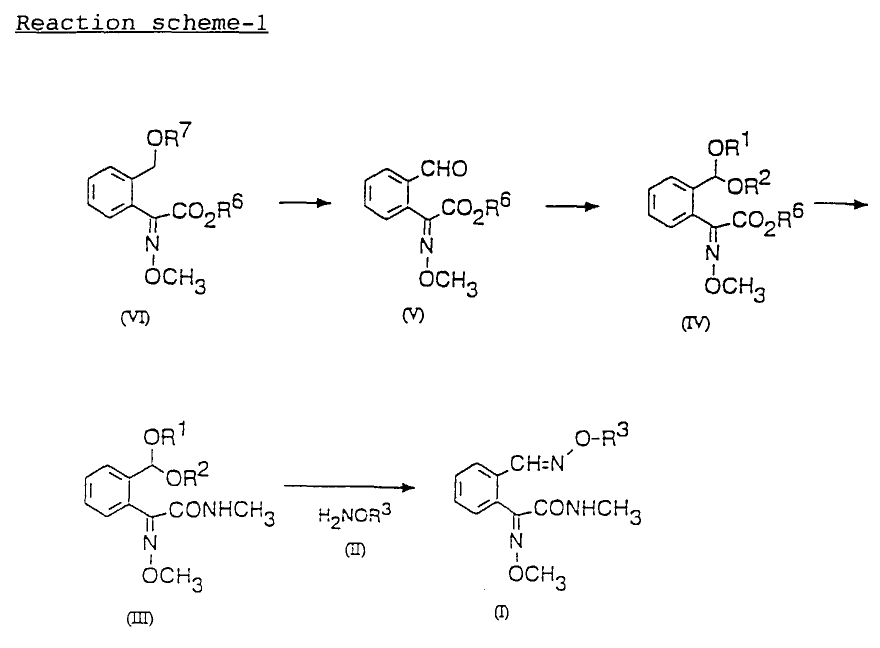 (In the above scheme, R¹, R² and R³ are as defined in the foregoing, R⁶ is a C₁-C₄ alkyl group and R⁷ is a C₁-C₄ alkyl group.)

Hereinafter, the present invention is described in detail.

In the above general formula (III), each of R¹ and R² represents a C₁-C₄ alkyl group, or R¹ and R² in combination represent a C₂-C₄ alkylene group. Preferably, it represents methyl, ethyl or ethylene.

In the above general formula (II), R³ represents a hydrogen atom or a group represented by a general formula -C(R⁴)(R⁵)-Ar. Each of R⁴ and R⁵ independently represents a hydrogen atom or a C₁-C₄ alkyl group such as methyl, ethyl, n-propyl, iso-propyl, n-butyl, sec-butyl or the like. Among these groups, each of R⁴ and R⁵ is preferably a hydrogen atom or a methyl group, independently.

Ar represents an aryl group (e.g., phenyl, naphthyl or the like) which may be substituted with a group exemplified in the following; or a heteroaryl group (e.g., pyridyl, thienyl, thiazolyl or the like) which may be substituted with a group exemplified in the following. Preferably, it represents a phenyl group which may be substituted with a group exemplified in the following.

Examples of the aforementioned substituent for the aryl group include a cyano group; a halogen atom (e.g., fluorine, chlorine, bromine or the like); a C₁-C₆ alkyl group (e.g., methyl, ethyl, n-propyl, iso-propyl, n-butyl, sec-butyl or the like); a C₂-C₄ alkenyl group (e.g., ethenyl, propenyl or the like) which may be substituted with a halogen atom; a C₁-C₄ haloalkyl group (e.g., trifluoromethyl, difluoromethyl, trichloromethyl, dichlorodifluoroethyl or the like); a C₁-C₆ alkoxy group (e.g., methoxy, ethoxy, iso-propoxy, n-butoxy or the like) which may be substituted with a halogen atom or a C₃-C₆ cycloalkyl group; a C₁-C₆ alkylthio group (e.g., methylthio, ethylthio, iso-propylthio, n-butylthio or the like); an aryl group (e.g., phenyl or the like) which may be substituted with a C₁-C₄ alkyl group or a halogen atom; an aryloxy group (e.g., phenoxy or the like) which may be substituted with a C₁-C₄ alkyl group or a halogen atom; a C₂-C₆ alkenyloxy group (e.g., propenyloxy or the like) which may be substituted with a halogen atom; and a C₂-C₆ alkynyloxy group (e.g., propargyloxy or the like). The alkyl, alkenyl or alkynyl moiety of these substituents may be either straight or branched chain. Among these substituents, preferable substituents for the aryl group include a C₁-C₄ alkyl group, a halogen atom, a C₁-C₄ alkoxy group which may be substituted with a halogen atom (preferably, fluorine), or a trifluoromethyl group. In addition, adjacent two substituents in combination represent a group such as methylenedioxy, ethylenedioxy or the like to form a fused ring with the aryl group.

The number of substituents is 1 to 5, preferably 1 or 2. When it has a plurality of substituents, they may be the same or different from one another.

Examples of the aforementioned substituent for the heteroaryl group include a cyano group; a halogen atom (e.g., fluorine, chlorine, bromine or the like); a C₁-C₆ alkyl group (e.g., methyl, ethyl, n-propyl, iso-propyl, n-butyl, sec-butyl or the like); a C₁-C₄ haloalkyl group (e.g., trifluoromethyl, difluoromethyl, trichloromethyl, dichlorodifluoroethyl or the like); and a C₁-C₆ alkoxy group (e.g., methoxy, ethoxy, iso-propoxy, n-butoxy or the like) which may be substituted with a halogen atom or a C₃-C₆ cycloalkyl group. The alkyl moiety of these substituents may be either straight or branched chain. Among these substituents, preferable substituents for the heteroaryl group include a C₁-C₄ alkyl group; a halogen atom; or a trifluoromethyl group. The number of substituents is 1 or 2 which may be the same or different from each other.

The N-methyl-methoxyiminoacetamide derivative (I) is produced by allowing an acetal derivative (III) to react with an oxyamine derivative (II) without solvent or in an inert solvent in the presence of an appropriate acid.

The oxyamine derivative (II) may be used as such or, as occasion demands, in the form of a salt (e.g., hydrochloride, sulfate or the like) in an amount of 1 to 5 equivalents, preferably 1 to 3 equivalent, based on the compound (III).

Examples of the acid to be used include organic acids such as acetic acid, trifluoroacetic acid and the like; halogenated hydroacids such as hydrochloric acid and the like; hydrogen halides such as hydrogen chloride and the like; sulfonic acids such as methanesulfonic acid, p-toluenesulfonic acid, sulfuric acid and the like; acid addition salts of organic bases such as pyridine hydrochloride, pyridine sulfate and the like; and Lewis acids such as zinc chloride, iron chloride, aluminum chloride and the like. Organic acids are preferred. These acids may be used in an amount of from catalytically effective amount to 10 equivalent, preferably from catalytically effective amount to 1 equivalent, based on the compound (III).

Examples of the solvent to be used include aromatic hydrocarbons such as benzene, toluene, xylene and the like; halogenated hydrocarbons such as dichloromethane, chloroform, 1,2-dichloroethane and the like; ethers such as diethyl ether, tetrahydrofuran, dioxane and the like; esters such as ethyl acetate and the like; ketones such as acetone, methyl ethyl ketone and the like; alcohols such as methanol, ethanol, propanol and the like; nitriles such as acetonitrile and the like; and polar solvents such as N,N-dimethylformamide, N-methylpyrrolidone, dimethyl sulfoxide, acetic acid, water and the like, which may be used alone or as a mixture thereof. Preferred of these solvents are ethers such as tetrahydrofuran, dioxane and the like; alcohols; polar solvents such as N,N-dimethylformamide, acetic acid and the like. The reaction can be carried out at a temperature of from room temperature to boiling point of the solvent used, preferably from 20 to 120°C. The reaction time is 30 minutes to 48 hours, preferably 1 to 24 hours.

The acetal derivative (III) to be used as the starting material in the production method of the present invention is a novel compound and can be produced, for example, by the method shown in the following reaction scheme-2. 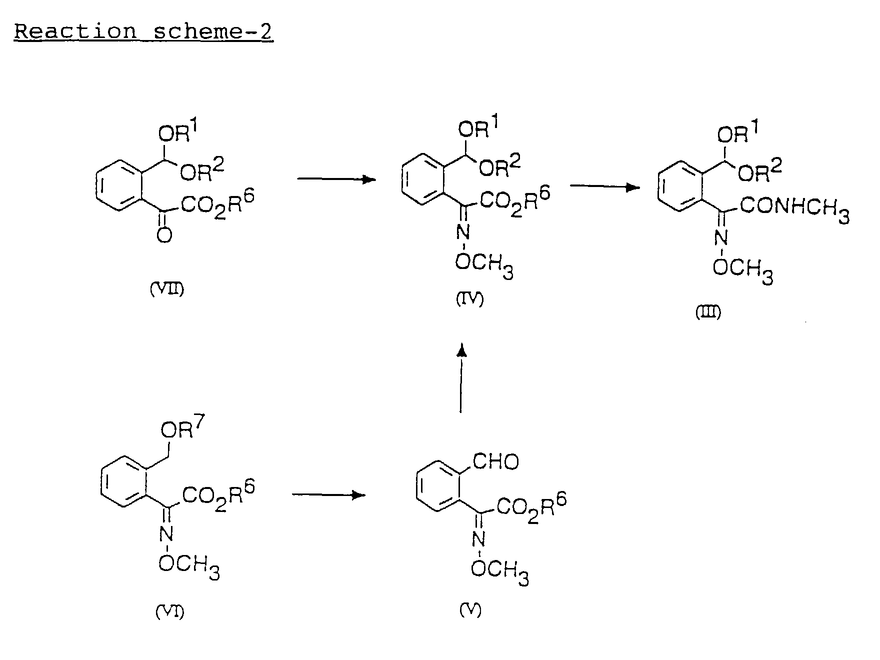 (In the above scheme, R¹, R², R⁶ and R⁷ are as defined in the foregoing.)

That is, the acetal derivative (III) can be produced by treating a methoxyiminoacetic acid derivative (IV) with methylamine preferably in an inert solvent. Methylamine may be used in an amount of from 1 equivalent to a large excess, preferably from 1 to 3 equivalents, based on the compound of formula (IV).

Examples of the solvent to be used include aromatic hydrocarbons such as benzene, toluene, xylene and the like; halogenated hydrocarbons such as dichloromethane, chloroform, 1,2-dichloroethane and the like; ethers such as diethyl ether, tetrahydrofuran, dioxane and the like; ketones such as acetone, methyl ethyl ketone and the like; alcohols such as methanol, ethanol, propanol and the like; and polar solvents such as N,N-dimethylformamide, N-methylpyrrolidone, dimethyl sulfoxide, acetonitrile, water and the like, which may be used alone or as a mixture thereof. Preferred of these solvents is toluene, tetrahydrofuran, methanol, ethanol or water. The reaction can be carried out at a temperature of from -78°C to boiling point of the solvent used, preferably selected in the range of from 0 to 60°C. The reaction time is about 30 minutes to 48 hours, preferably about 1 to 24 hours.

The methoxyiminoacetic acid derivative (IV) can be produced by subjecting a glyoxylic acid ester derivative (VII) to oxime formation reaction by a known method (for example, a method described in an unexamined published Japanese patent application (*kokai*) No. 5-97768) or a modified method thereof, or by subjecting an aldehyde derivative (V) to acetal formation reaction by a known method (for example, a method described in *Synthetic Communication*, 7, 409 (1977)) or a modified method thereof.

The former method by way of the glyoxilic acid ester derivative (VII) requires 2-bromobenzaldehyde to be used as the starting material which is extremely expensive. Thus, this production method is not considered to be an economically satisfactory. On the other hand, the latter method by way of the aldehyde derivative (V) is an industrially advantageous production method since inexpensive 2-methoxymethylchlorobenzene can be used as the starting material.

A method in which a benzal halide derivative is treated with an oxidizing agent such as silver nitrate or the like (for example, a method described in *J. Am. Chem. Soc*., 78, 1689 (1956)) or a method in which a benzyl halide derivative is oxidized using N-methylmorpholine-N-oxide (for example, a method described in an unexamined published Japanese patent application (*kokai*) No. 6-25132) is hitherto known as the production method for the aldehyde derivative (V). However, neither of these method can be regarded as an industrially satisfactory method, because not only the oxidizing agents to be used are extremely expensive but also they have a possibility of causing explosion at a high temperature.

The present inventors have found that the aldehyde derivative (V) can be produced easily by treating an alkoxymethyl derivative (VI) with a halogenation agent without solvent or in an inert solvent. In that case, the derivative can be produced stably with a high yield by allowing an appropriate base to coexist. The method in which the aldehyde derivative (V) is produced from the alkoxymethyl derivative (VI) is also included in the present invention.

Examples of R⁶ and R⁷ of the general formula (VI) independently include a C₁-C₄ straight or branched alkyl group such as methyl, ethyl, n-propyl, i-propyl, n-butyl, t-butyl and the like, of which methyl, ethyl or t-butyl is preferred.

Examples of the halogenation agent to be used include chlorine, bromine, iodine, sulfuryl chloride, sulfuryl bromide, N-chlorosuccinimide, N-bromosuccinimide, 1,3-dibromo-5,5-dimethylhydantoin and the like, preferably chlorine or bromine. The halogenation agent is used in an amount of from 1 equivalent to 5 equivalents, preferably from 1 equivalent to 2 equivalents, based on the alkoxymethyl derivative (VI).

Examples of the base to be used include alkali metal bicarbonates such as sodium bicarbonate and the like; alkali metal carbonates such as sodium carbonate and the like; alkaline earth metal carbonates such as calcium carbonate and the like; alkali metal acetates such as sodium acetate and the like; alkaline earth metal acetates such as calcium acetate and the like; organic bases such as pyridine, triethylamine and the like; and polymeric bases such as polyvinyl pyrrolidone (cross-linked), polyvinyl pyridine and the like, preferably sodium bicarbonate or sodium acetate. The base is used in an amount of from 1 equivalent to 5 equivalents, preferably from 2 equivalents to 3 equivalents, based on the alkoxymethyl derivative (VI).

Examples of the solvent to be used include hydrocarbons such as n-hexane, cyclohexane and the like; aromatic hydrocarbons such as benzene, toluene, xylene and the like; halogenated hydrocarbons such as dichloromethane, chloroform, 1,2-dichloroethane and the like; ethers such as diethyl ether, tetrahydrofuran, dioxane and the like; esters such as ethyl acetate and the like; ketones such as acetone, methyl ethyl ketone and the like; alcohols such as methanol, ethanol, propanol and the like; nitriles such as acetonitrile and the like; and polar solvents such as N,N-dimethylformamide, N-methylpyrrolidone, dimethyl sulfoxide, pyridine, acetic acid, water and the like, which may be used alone or as a mixture thereof. Preferred of these are alcohols and polar solvents such as N,N-dimethylformamide, acetic acid and the like.

The reaction can be carried out at a temperature of from room temperature to boiling point of the solvent used, preferably from 20 to 120°C.

Though the reaction method is not particularly limited, it is desirable to carry out the reaction by gradually adding the halogenation agent.

The reaction time is about 1 hour to 48 hours, preferably about 2 to 24 hours.

As shown in the following reaction scheme-3, the alkoxymethyl derivative (VI) is produced by first producing a glyoxylic acid ethyl ester derivative from 2-methoxymethylchlorobenzene by means of a known method (for example, a method described in *Synthetic Communication*, 11, 943 (1981)) or a modified method thereof and then subjecting the resulting derivative to oxime formation reaction by a known method (for example, a method described in an unexamined published Japanese patent application (*kokai*) No. 5-97768) or a modified method thereof. 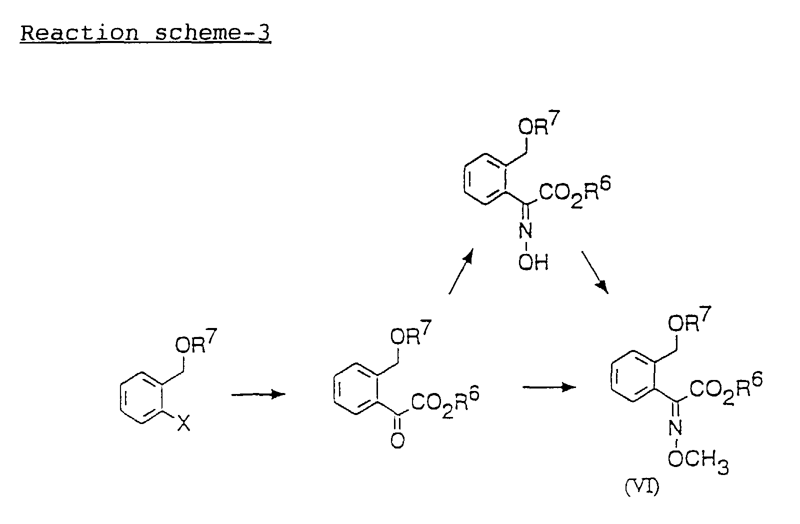 (In the above scheme, X is a halogen atom such as chlorine, bromine, iodine or the like, and R⁶ and R⁷ are as defined in the foregoing.)

Since the resulting alkoxymethyl derivative (VI) has isomers (E and Z) due to the asymmetric carbon atom to which the methoxyimino group is linked, a reaction mixture is generally obtained as a mixture of these isomers, and the Z isomer can be converted into E isomer by treating it or the mixture with an acid (hydrochloric acid or the like) in an alcohol solvent. When the E isomer is used as the starting material, the N-methyl-methoxyiminoacetamide derivative of formula (I) can finally be obtained as its E isomer which is desirable from the viewpoint of biological activity.

In the production method for the N-methyl-methoxyiminoacetamide derivative of formula (I), a compound represented by H₂NO-CH(R⁴)-Ar (wherein R⁴ and Ar are as described in the foregoing) of a general formula (II') as a member of the oxyamine derivative (II) to be used as the starting material can be produced, for example, in accordance with the following reaction scheme-4. 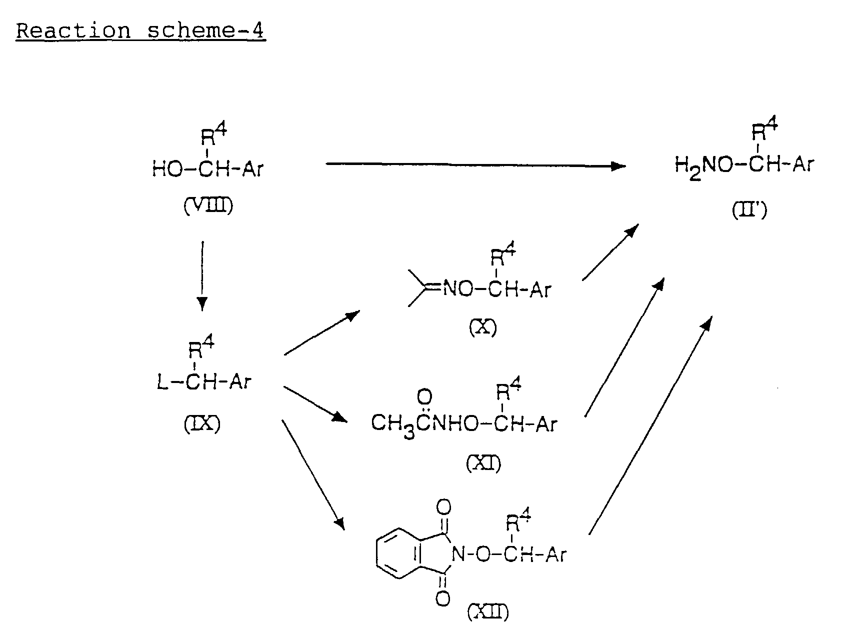 (In the above scheme, R⁴ and Ar are as described in the foregoing, and L is a leaving group.)

That is, the oxyamine derivative (II) can be produced directly by allowing its corresponding benzyl alcohol (VIII) to react with chloramine (*Angew. Chem.*, 68, 303 (1956), *Angew. Chem.*, 72, 127 (1960)).

Also, the oxyamine derivative (II') can be produced by acid hydrolysis of an acetoxime derivative (X) which is obtained from its corresponding benzyl alcohol (VIII) through a compound (IX) having a leaving group such as a chlorine atom, a methanesulfonyl group or the like. (*Tetrahedron*, 23, 4441 (1967), *Org. Synth. Coll* Vol. III, 172 (1955)). It can also be obtained by acid hydrolysis of an acetohydroxamic acid derivative (XI) which can be obtained in the same manner as described in the foregoing. (*J. Chem. Soc.*, 3127, (1963), *J. Chem. Soc.*, 1632, (1958)). It can also be produced by treating an oxyphthalimide (XII) with hydrazine hydrate (*J. Org. Chem.*, 36, 24, 3835 (1971)).

### BEST MODE FOR CARRYING OUT THE INVENTION

The present invention is further illustrated by way of the following Examples. The present invention is not limited to the following Examples as long as it does not exceed the gist of the invention.

### Example 1

### Synthesis of ethyl 2-(2-formylphenyl)-2-methoxyiminoacetate (V)

To a mixture of 4.0 g (15.9 mmol) of ethyl 2-(2-methoxymethylphenyl)-2-methoxyiminoacetate, 3.14 g (38.2 mmol: 2.4 eq) of anhydrous sodium acetate and 16 ml of acetic acid was added dropwise a mixture of 2.80 g (17.5 mmol: 1.1 eq) of bromine and 4 ml of acetic acid, spending 10 minutes, and the resulting mixture was stirred at 80°C for 4 hours and then spontaneously cooled by allowing it to stand for 1.5 hours. After adding 100 ml of toluene, the reaction mixture was washed with water, saturated sodium bicarbonate aqueous solution and saturated brine in that order and then dried over anhydrous sodium sulfate. After evaporation of the solvent, the residue was purified by silica gel column chromatography (hexane/toluene/ethyl acetate = 6:1:1 - 3:1:1) to give 2.32 g of the title compound as white crystals. The yield was 61.9%.
Melting point; 96.0 - 96.5°C
¹H-NMR δ (CDCl₃); 1.34 (3H, t), 4.03 (3H, s), 4.35 (2H, q), 7.35 (1H, d), 7.60 (1H, dd), 7.68 (1H, dd), 7.94 (1H, d), 9.90 (1H, s)

### Example 2

### Synthesis of ethyl 2-(2-formylphenyl)-2-methoxyiminoacetate (V)

A mixture of 2.0 g (8.00 mmol) of ethyl 2-(2-methoxymethylphenyl)-2-methoxyiminoacetate, 1.50 g (18.3 mmol: 2.3 eq) of anhydrous sodium acetate and 4 ml of acetic acid was heated at 80°C, and a mixture of 1.40 g (8.76 mmol: 1.1 eq) of bromine and 6 ml of acetic acid was added dropwise spending 80 minutes. The mixture was stirred for 4 hours and then allowed to stand overnight. After adding 100 ml of toluene, the reaction mixture was washed with water, saturated sodium bicarbonate aqueous solution and saturated brine in that order and then dried over anhydrous sodium sulfate. After evaporation of the solvent, the residue was purified by silica gel column chromatography (silica gel 100 g, hexane/toluene/ethyl acetate = 6:1:1 - 5:1:1) to give 1.34 g of the title compound as white crystals. The yield was 71.5%. Measurement of its NMR spectrum confirmed that it was the same compound as Example 1.

### Example 3

### Synthesis of N-methyl-2-(2-diethoxymethylphenyl)-2-methoxyiminoacetamide (III)

A mixture of 10.0 g (42.6 mmol) of ethyl 2-(2-formylphenyl)-2-methoxyiminoacetate, 9.5 g (93.8 mmol) of triethyl orthoformate, 0.08 g (0.4 mmol) of p-toluenesulfonic acid, 40 ml of ethanol and 100 ml of toluene was heated under reflux for 4 hours. After evaporation of solvent from the reaction mixture, the resulting residue was purified by silica gel column chromatography (ethyl acetate/hexane = 1:5) to give 12.5 g of ethyl 2-(2-diethoxymethylphenyl)-2-methoxyiminoacetate. The yield was 95%.
¹H-NMR δ (CDCl₃); 1.18 (6H, t), 1.32 (3H, t), 3.49 (4H, q), 4.01 (3H, s), 4.32 (2H, q), 5.39 (1H, s), 7.13 (1H, d), 7.37 (1H, dd), 7.41 (1H, dd), 7.66 (1H, d)

A mixture of 12.1 g (39.2 mmol) of ethyl 2-(2-diethoxymethylphenyl)-2-methoxyiminoacetate, 50 ml of methanol and 50 ml of 40% methylamine solution in methanol was stirred at room temperature for 4 hours. After evaporation of solvent from the reaction mixture, hexane was added, and the crystals precipitated were collected by filtration to give 10.3 g of the title compound. The yield was 89%.
Melting point; 75.5 - 76.0°C
¹H-NMR δ (CDCl₃); 1.18 (6H, br), 2.91 (3H, d), 3.46 (4H, br), 3.94 (3H, s), 5.44 (1H, s), 7.14 (1H, d), 7.37 (1H, dd), 7.41 (1H, dd), 7.68 (1H, d)

### Example 4

### Synthesis of N-methyl-2-{2-(1,3-dioxolan-2-yl)phenyl}-2-methoxyiminoacetamide (III)

A mixture of 50.0 g (0.270 mol) of 2-bromobenzaldehyde, 33.5 g (0.54 mol) of ethylene glycol, 0.51 g (2.7 mmol) of p-toluenesulfonic acid and 300 ml of toluene was heated under reflux for 2 hours while removing water. The reaction mixture was cooled and 300 ml of toluene was added. The mixture was washed with saturated sodium bicarbonate aqueous solution, water and saturated brine in that order and then dried over anhydrous sodium sulfate. After evaporation of the solvent, the residue was distilled under a reduced pressure to give 66.0 g of 2-(2-bromophenyl)-1,3-dioxolan. The yield was 94%.
Boiling point; 104 - 108°C/1.5 mm
¹H-NMR δ (CDCl₃); 4.05 - 4.2 (4H, m), 6.11 (1H, s), 7.2 - 7.6 (4H, m)

A 140 ml THF solution containing 51.0 g (223 mmol) of 2-(2-bromophenyl)-1,3-dioxolan was added dropwise to a mixture of 5.41 g (270 mmol) of magnesium and 100 ml of tetrahydrofuran (THF) spending 30 minutes, and the mixture was further stirred for 45 minutes. The resulting Grignard reagent was added dropwise to a mixture of 65.0 g (445 mmol) of diethyl oxalate and 1,000 ml of ether, at an inner temperature of -30 to -20°C spending 15 minutes. After 3 hours of stirring at -20°C to 0°C, the reaction mixture was poured into 500 ml of saturated ammonium chloride aqueous solution to effect separation, and the resulting organic layer was washed with water and saturated brine and dried over anhydrous sodium sulfate. After evaporation of the solvent, excess diethyl oxalate was evaporated under a reduced pressure (55 - 65°C/1.5 mm), and the resulting residue (about 46 g) was purified by silica gel column chromatography (ethyl acetate/hexane = 1:4) to give 40.0 g of ethyl 2-(1,3-dioxolan-2-yl)phenylglyoxylate. The yield was 72%.
¹H-NMR δ (CDCl₃); 1.38 (3H, t), 3.8 - 4.0 (4H, m), 4.38 (2H, q), 6.25 (1H, s), 7.45 (1H, dd), 7.51 (1H, d), 7.55 (1H, dd), 7.60 (1H, d)
¹³C-NMR δ (CDCl₃); 14.0, 62.4, 64.7, 101.7, 126.7, 128.9, 129.4, 131.9, 134.4, 139.0, 161.8, 188.2

To a mixture of 12.4 ml (153 mmol) of pyridine and 70 ml of ethanol was added 10.2 g (122 mmol) of methoxyamine hydrochloride, and the mixture was stirred at room temperature for 20 minutes. Then, 60 ml of an ethanol solution containing 25.5 g (102 mmol) of ethyl 2-(1,3-dioxolan-2-yl)phenylglyoxylate was added to the mixture and stirred at room temperature for 3 hours. After evaporation of the solvent from the reaction mixture, 400 ml of ethyl acetate was added. The mixture was washed with water and saturated brine and then dried over anhydrous sodium sulfate. After evaporation of the solvent, the resulting residue was purified by silica gel column chromatography (ethyl acetate/hexane = 1:5) to give 16.8 g of ethyl 2-{2-(1,3-dioxolan-2-yl)phenyl}-2-methoxyiminoacetate (IV). The yield was 53%.
Melting point; 84.0 - 85.0°C
¹H-NMR δ (CDCl₃); 1.31 (3H, t), 3.9 - 4.0 (4H, m), 4.02 (3H, s), 4.33 (2H, q), 5.78 (1H, s), 7.16 (1H, d), 7.40 (1H, dd), 7.43 (1H, dd), 7.60 (1H, d)
¹³C-NMR δ (CDCl₃); 14.1, 61.9, 63.6, 65.0, 102.6, 126.9, 128.76, 128.83, 129.1, 129.8, 136.0, 150.5, 163.1

A mixture of 9.5 g (34.1 mmol) of ethyl 2-{2-(1,3-dioxolan-2-yl)phenyl}-2-methoxyiminoacetate and 40 ml of 40% methylamine solution in methanol was stirred at room temperature for 4 hours. After evaporation of the solvent from the reaction mixture, hexane was added, and the crystals precipitated were collected by filtration to give 8.86 g of the title compound. The yield was 99%.
Melting point; 110.0 - 111.5°C
¹H-NMR δ (CDCl₃); 2.93 (3H, d), 3.95 (7H, s), 5.88 (1H, s), 6.73 (1H, br), 7.17 (1H, d), 7.36 - 7.46 (2H, m), 7.61 (1H, d)

### Example 5

### Synthesis of N-methyl-2-(2-hydroxyliminomethylphenyl)-2-methoxyiminoacetamide (I)

A mixture of 1.0 g (3.8 mmol) of N-methyl-2-{2-(1,3-dioxolan-2-yl)phenyl}-2-methoxyiminoacetamide, 0.40 g (5.8 mmol) of hydroxylamine hydrochloride, 1.0 g (7.6 mmol) of sodium acetate trihydrate and 5 ml of acetic acid was stirred at 60°C for 4 hours. After evaporation of the solvent from the reaction mixture, water was added, and the mixture was extracted with ethyl acetate. The organic layer was washed with water, saturated sodium bicarbonate aqueous solution and saturated brine in that order and then dried over anhydrous sodium sulfate. After evaporation of the solvent, the residue was purified by silica gel column chromatography (ethyl acetate/hexane = 1:1) to give 0.64 g of the title compound in the form of white crystals. The yield was 72%.
Melting point; 192.5 - 197.5°C
¹H-NMR δ (CDCl₃); 2.91 (3H, d), 3.94 (3H, s), 6.98 (1H, br), 7.16 - 7.22 (1H, m), 7.36 - 7.42 (2H, m), 7.80 - 7.86 (1H, m), 7.91 (1H, s)

### Example 6

### Synthesis of N-methyl-2-{2-(1-phenylethoxyiminomethyl)phenyl}-2-methoxyiminoacetamide (I)

A mixture of 1.0 g (3.8 mmol) of N-methyl-2-{2-(1,3-dioxolan-2-yl)phenyl}-2-methoxyiminoacetamide, 0.57 g (4.2 mmol) of 1-phenylethoxyamine and 5 ml of acetic acid was stirred at 60°C for 4 hours. After evaporation of the solvent from the reaction mixture, water was added, and the mixture was extracted with ethyl acetate. The organic layer was washed with water, saturated sodium bicarbonate aqueous solution and saturated brine in that order and then dried over anhydrous sodium sulfate. After evaporation of the solvent, the residue was purified by silica gel column chromatography (ethyl acetate/hexane = 1:2) to give 0.71 g of the title compound in the form of white crystals. The yield was 55%.
Melting point; 107.5 - 110.2°C
¹H-NMR δ (CDCl₃); 1.58 (3H, d), 2.86 (3H, d), 3.87 (3H, s), 5.26 (1H, q), 6.56 (1H, br), 7.16 (1H, m), 7.3 - 7.4 (7H, m), 7.68 (1H, m), 7.96 (1H, s)

### Example 7

### Synthesis of N-methyl-2-[2-{1-(4-difluoromethoxy-3-methoxyphenyl)ethoxyiminomethyl}phenyl]-2-methoxyiminoacetamide (I)

A mixture of 1.0 g (3.8 mmol) of N-methyl-2-{2-(1,3-dioxolan-2-yl)phenyl}-2-methoxyiminoacetamide, 0.97 g (4.2 mmol) of 1-(4-difluoromethoxy-3-methoxyphenyl)ethoxyamine, 2.5 ml of THF and 2.5 ml of acetic acid was stirred at 60°C for 4 hours. After evaporation of the solvent from the reaction mixture, water was added, and the mixture was extracted with ethyl acetate. The organic layer was washed with water, saturated sodium bicarbonate aqueous solution and saturated brine in that order and then dried over anhydrous sodium sulfate. After evaporation of the solvent, the residue was purified by silica gel column chromatography (ethyl acetate/hexane = 1:2) to give 1.03 g of the title compound. The yield was 63%.
¹H-NMR δ (CDCl₃); 1.57 (3H, d), 2.88 (3H, d), 3.88 (3H, s), 3.89 (3H, s), 5.23 (1H, q), 6.54 (1H, t), 6.65 (1H, br), 6.91 (1H, dd), 6.97 (1H, d), 7.1 - 7.2 (2H, m), 7.35 - 7.4 (2H, m), 7.70 (1H, m), 7.96 (1H, s)

### Example 8

### Synthesis of N-methyl-2-[2-{1-(4-chlorophenyl)ethoxyiminomethyl}phenyl]-2-methoxyiminoacetamide (I)

A mixture of 0.80 g (3.0 mmol) of N-methyl-2-{2-(1,3-dioxolan-2-yl)phenyl}-2-methoxyiminoacetamide, 0.57 g (0.33 mmol) of 1-(4-chlorophenyl)ethoxyamine, 0.2 ml of water and 4 ml of acetic acid was stirred at 60°C for 4 hours. After evaporation of the solvent from the reaction mixture, water was added, and the mixture was extracted with ethyl acetate. The organic layer was washed with water, saturated sodium bicarbonate aqueous solution and saturated brine in that order and then dried over anhydrous sodium sulfate. After evaporation of the solvent, the residue was purified by silica gel column chromatography (ethyl acetate/hexane = 1:1) to give 0.80 g of the title compound in the form of white crystals. The yield was 71%.
Melting point; 87.5 - 90.5°C
¹H-NMR δ (CDCl₃); 1.56 (3H, d), 2.83 (3H, d), 3.87 (3H, s), 5.26 (1H, q), 6.76 (1H, br, s), 7.17 (1H, dd), 7.26 - 7.40 (6H, m), 7.70 (1H, dd), 7.98 (1H, s)

### Example 9

### Synthesis of N-methyl-2-[2-{1-(3-trifluoromethylphenyl)ethoxyiminomethyl}phenyl]-2-methoxyiminoacetamide (I)

A mixture of 2.0 g (6.8 mmol) of N-methyl-2-(2-diethoxymethylphenyl)-2-methoxyiminoacetamide, 0.53 g (7.5 mmol) of 1-(3-trifluoromethylphenyl)ethoxyamine, 0.2 ml of water and 4 ml of acetic acid was stirred at 60°C for 4 hours. After evaporation of the solvent from the reaction mixture, water was added, and the mixture was extracted with ethyl acetate. The organic layer was washed with water, saturated sodium bicarbonate aqueous solution and saturated brine in that order and then dried over anhydrous sodium sulfate. After evaporation of the solvent, the residue was purified by silica gel column chromatography (ethyl acetate/hexane = 1:1) to give 2.52 g of the title compound in the form of white crystals. The yield was 88%.
Melting point; 88.5 - 89.0°C
¹H-NMR δ (CDCl₃); 1.58 (3H, d), 2.88 (3H, d), 3.86 (3H, s), 5.30 (1H, q), 6.67 (1H, br), 7.15 (1H, dd), 7.35 - 7.55 (5H, m), 7.60 (1H, s), 7.68 (1H, dd), 7.96 (1H, s)

In the same manner as described in Examples 5 to 9, the following compounds shown in Tables 1 and 2 can be produced.

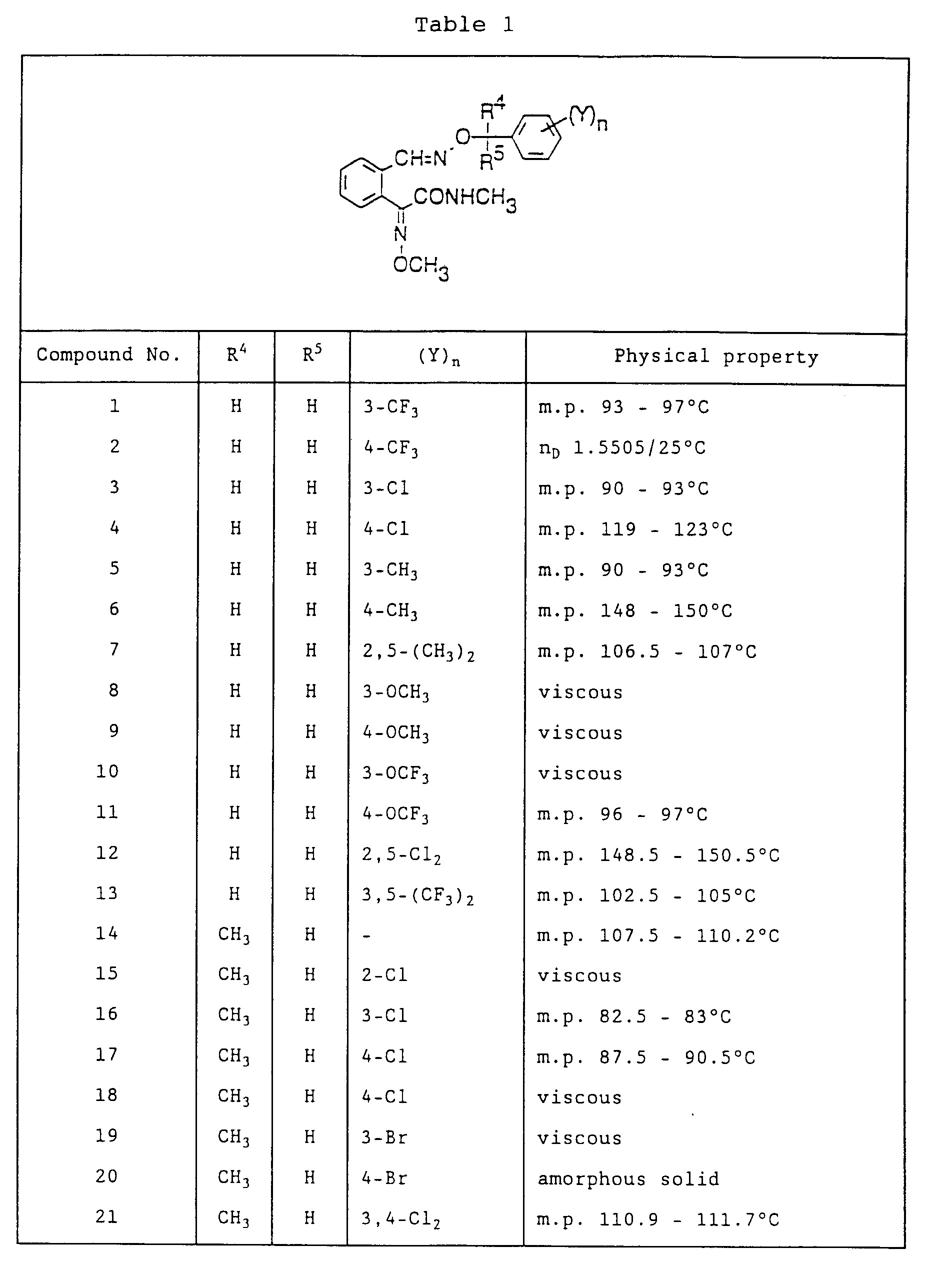

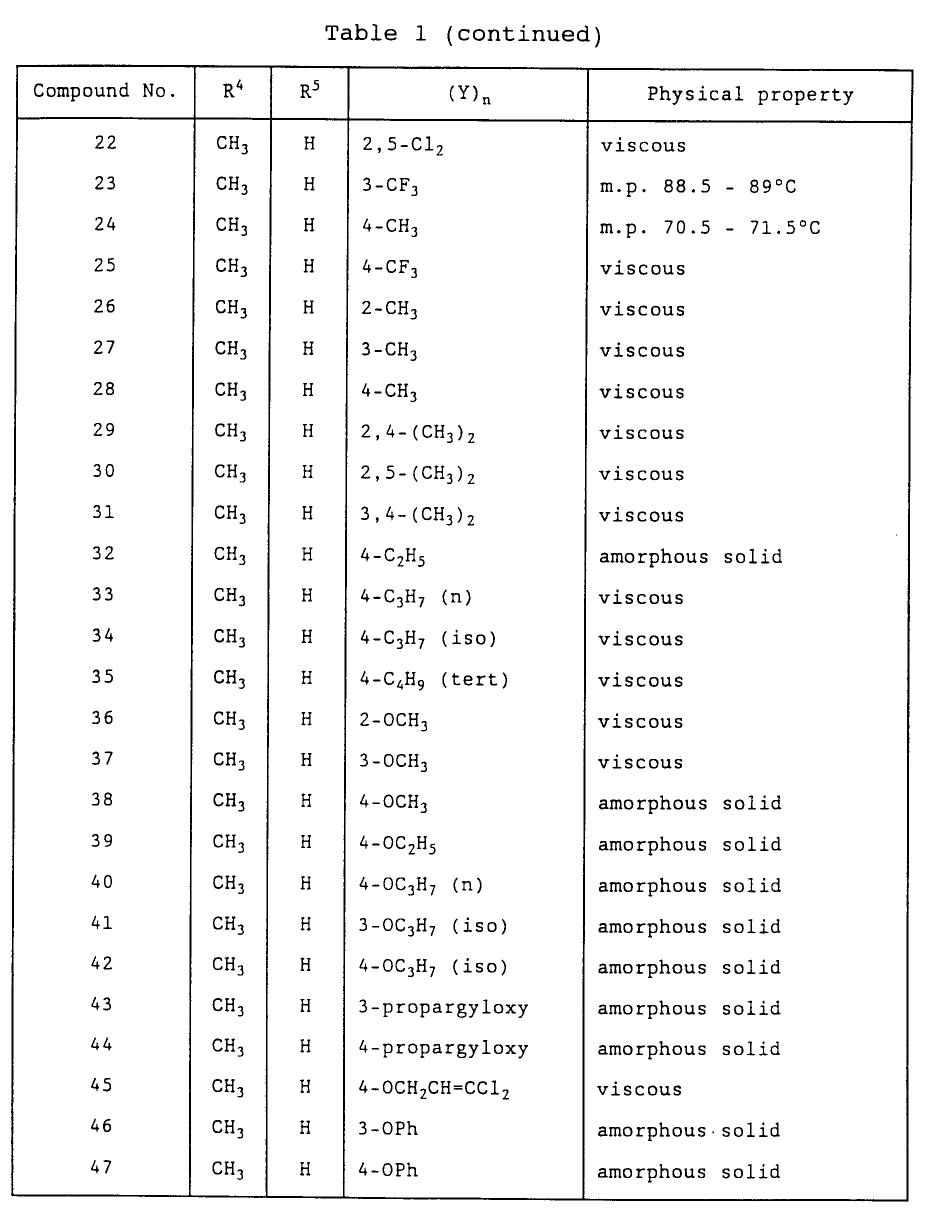

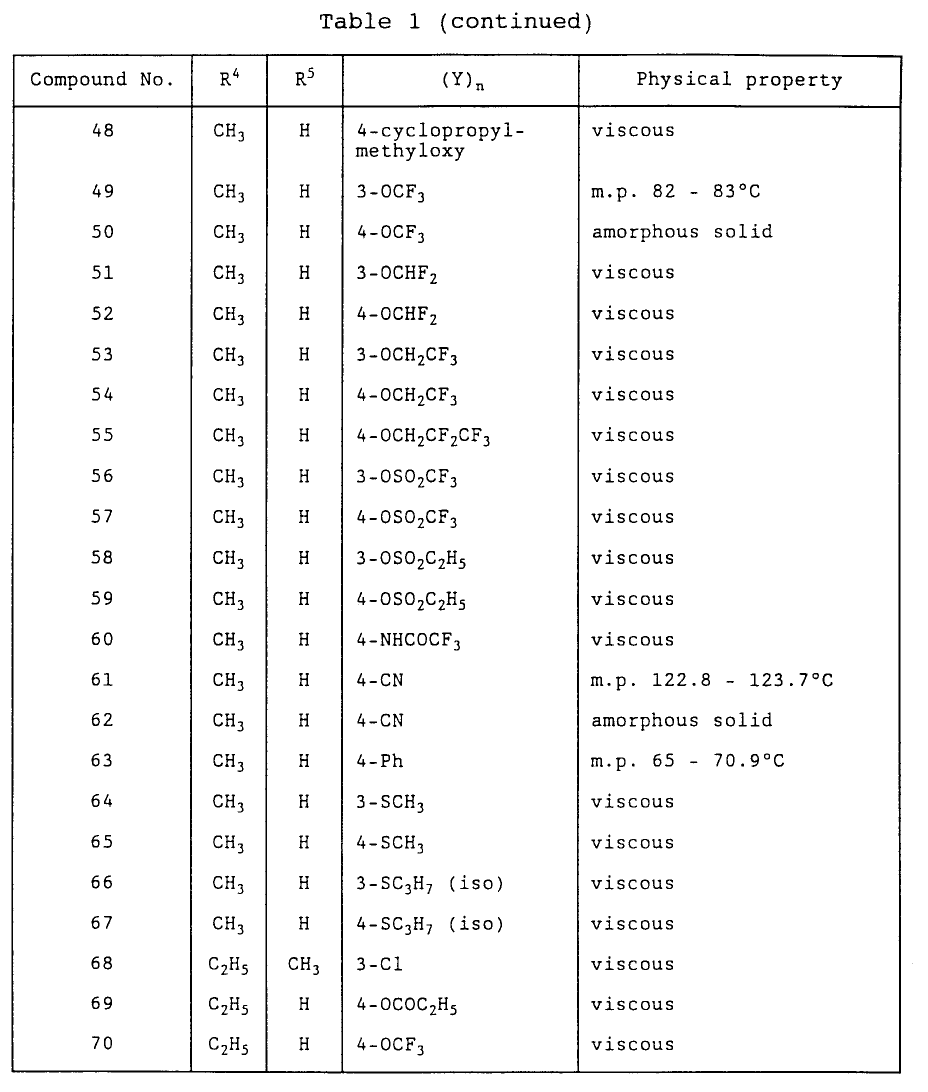

- *: The compound Nos. 18, 25 and 62 are isomers of compound Nos. 17, 24 and 61 in the benzyloxyimino moiety, respectively.
- **: Though E-form and Z-form isomers exist in the methoxyimino moiety, physical properties of only E-form are shown in Tables 1 and 2, unless otherwise noted.

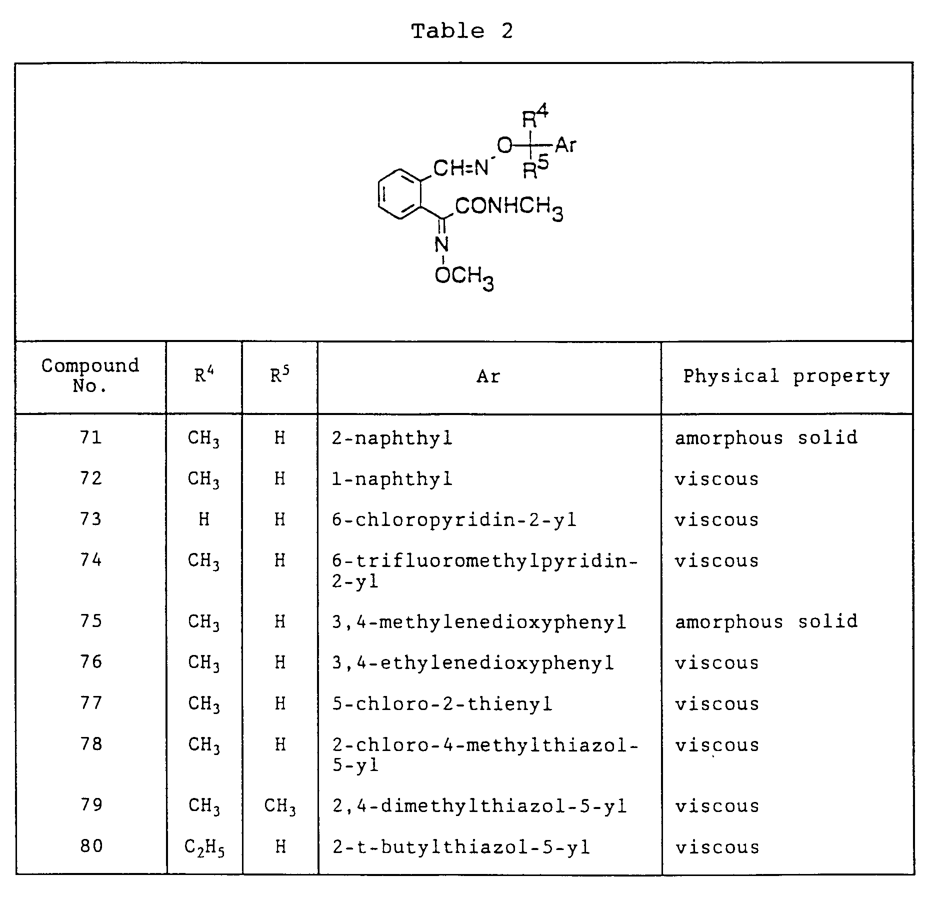

Production examples for the starting materials for the present invention are described in the following.

### Reference Example 1

### Synthesis of ethyl 2-methoxymethylphenyl-2-methoxyiminoacetate (VI)

To a mixture of 15.0 g (67.6 mmol) of 2-methoxymethylphenylglyoxylate, 6.65 g (81.1 mmol, 1.2 eq) of anhydrous sodium acetate and 100 ml of ethanol was added 6.77 g (81.1 mmol: 1.2 eq) of methoxyamine hydrochloride, and the mixture was stirred at room temperature for 19 hours. After adding 100 ml of toluene, the reaction mixture was filtered, the solvent was evaporated from the resulting filtrate, and then the residue was purified by silica gel column chromatography (silica gel 200 g, hexane/ethyl acetate = 10:1 - 5:1) to give 9.29 g of E/Z mixture, and 6.94 g of E-isomer, of the title compound (yield 95.6%).

A 4.4 g (37.0 mmol) of thionyl chloride was added dropwise to a mixture of 9.29 g (37.0 mmol) of the E/Z mixture of the title compound and 50 ml of ethanol, and the mixture was stirred at 60 to 70°C for 8 hours and then allowed to stand overnight. After evaporation of the solvent from the reaction mixture, 150 ml of ethyl acetate was added to the residue. The mixture was washed with water, saturated sodium bicarbonate aqueous solution and saturated brine in that order and then dried over anhydrous sodium sulfate. After evaporation of the solvent, the resulting residue was purified by silica gel column chromatography (silica gel 150 g, hexane/ethyl acetate = 10:1 - 4:1) to give 7.86 g of E-isomer of the title compound (total yield 14.8 g, yield 87.2%).

### (E-form)

¹H-NMR δ (CDCl₃); 1.32 (3H, t), 3.28 (3H, s), 4.03 (3H, s), 4.33 (2H, q), 4.33 (2H, s), 7.16 (1H, d), 7.32 - 7.46 (3H, m)

### (Z-form)

¹H-NMR δ (CDCl₃); 1.34 (3H, t), 3.43 (3H, s), 4.03 (3H, s), 4.36 (2H, q), 4.66 (2H, s), 7.28 - 7.46 (3H, m), 7.60 (1H, d)

### Reference Example 2

### Synthesis of ethyl 2-methoxymethylphenylglyoxylate

To a mixture of 12.4 g (511 mmol) of magnesium, 8.0 g of 2-methoxymethylchlorobenzene and 100 ml of tetrahydrofuran was added with heating under reflux and dropwise a mixture of 32.0 g (total 40.0 g, 256 mmol) of 2-methoxymethylchlorobenzene, 51.7 g (256 mmol) of 1,2-dibromoethane and 100 ml of tetrahydrofuran, spending 100 minutes. After further adding 100 ml of tetrahydrofuran, the mixture was heated under reflux for 1 hour, cooled and then added dropwise to a mixture of 74.6 g (511 mmol) of diethyl oxalate and 500 ml of tetrahydrofuran, spending 40 minutes at -20°C. The reaction mixture was further stirred at -20 to 0°C for 40 minutes and poured into saturated ammonium chloride aqueous solution to effect separation, and the organic layer was washed with saturated brine and dried over anhydrous sodium sulfate. After evaporation of the solvent, the resulting residue was purified by silica gel column chromatography (hexane/ethyl acetate = 6:1) to give 24.83 g of the title compound (yield 43.7%).
¹H-NMR δ (CDCl₃); 1.41 (3H, t), 3.40 (3H, s), 4.41 (2H, q), 4.76 (2H, s), 7.36 - 7.42 (1H, m), 7.54 - 7.60 (2H, m), 7.67 (1H, d)

### Reference Example 3

### Synthesis of 2-methoxymethylchlorobenzene

Spending 80 minutes, 100 ml of a methanol solution containing 161 g (1.0 mol) of 2-chlorobenzyl chloride was added dropwise to a mixture of 90 g (1.5 mol) of 90% sodium methoxide and 850 ml of methanol with heating under reflux. The reaction mixture was heated under reflux for 4 hours, the reaction solution was cooled and subjected to decantation to remove the solvent. Then, 500 ml of water was added to the residue, and the mixture was extracted with 1,000 ml and 500 ml of toluene. The organic layer was washed with water and saturated brine and dried over anhydrous sodium sulfate. The solvent was evaporated, and then the residue was distilled under a reduced pressure to give 140.48 g of the title compound.
Yield; 89.7%
Boiling point; 64 - 67°C/2.0 mm
¹H-NMR δ (CDCl₃); 3.46 (3H, s), 4.56 (2H, s), 7.18 - 7.32 (2H, m), 7.35 (1H, d), 7.46 (1H, d)

### Reference Example 4

### Synthesis of 3-trifluoromethyl-1'-chloroethylbenzene

Spending 1 hour, 186 g (1.56 mol) of thionyl chloride was added dropwise to 270 g (1.42 mol) of 1-(3-trifluoromethylphenyl)ethanol under ice-cooling. The reaction mixture was stirred for additional 1 hour, and 2,000 ml of ether was added. The mixture was washed with water, saturated sodium bicarbonate and saturated brine in that order and then dried over anhydrous magnesium sulfate. By evaporating the solvent, 297.2 g (quantitative) of the title compound was obtained.
¹H-NMR δ (ppm); 1.87 (3H, d), 5.12 (1H, q), 7.3 - 7.7 (4H, m)

### Reference Example 5

### Synthesis of 1-(3-trifluoromethylphenyl)ethoxyimino-2-propane

To a mixture of 2.09 g (10 mmol) of 3-trifluoromethyl-1'-chloroethylbenzene and 10 ml of dimethylformamide (DMF) was added 1.46 g (20 mmol) of acetone oxime and 2.76 g (20 mmol) of potassium carbonate, and the mixture was stirred at 150°C for 5 hours. After cooling to room temperature, ethyl acetate was added, and the mixture was washed with water and saturated brine and then dried over anhydrous sodium sulfate. The solvent was evaporated and the residue was purified by silica gel column chromatography (silica gel 50 g, hexane/ethyl acetate = 20:1) to give 1.33 g of the title compound. The yield was 54%.
¹H-NMR δ (ppm); 1.51 (3H, d), 1.83 (3H, s), 1.93 (3H, s), 5.22 (1H, q), 7.4 - 7.6 (4H, m)

### Reference Example 6

### Synthesis of 1-(3-trifluoromethylphenyl)ethoxyimino-2-propane

To a mixture of 0.59 g (10 mmol) of acetone oxime and 10 ml of DMF was added 1.12 g (10 mmol) of potassium t-butoxide under ice-cooling, and the mixture was stirred at room temperature for 20 minutes. Then, 2.09 g (10 mmol) of 3-trifluoromethyl-1'-chloroethylbenzene was added under ice-cooling, and the mixture was stirred at room temperature for 30 minutes and then at 80°C for 4 hours, followed by cooling to room temperature. Then, ethyl acetate was added, and the mixture was washed with water and saturated brine, and then dried over anhydrous sodium sulfate. The solvent was evaporated and the residue was purified by silica gel column chromatography (silica gel 50 g, hexane/ethyl acetate = 20:1) to give 1.54 g of the title compound (yield 63%).

### Reference Example 7

### Synthesis of 1-(3-trifluoromethylphenyl)ethoxyamine

A mixture of 0.49 g (2 mmol) of 1-(3-trifluoromethylphenyl)ethoxyimino-2-propane, ethanol (10 ml) and 5 ml of 5 N sulfuric acid aqueous solution was heated under reflux and 10 ml of the solvent was evaporated. Then, 10 ml of ethanol was added, and 10 ml of the solvent was evaporated in the same manner. Water was added to the reaction mixture, and the mixture was adjusted to a basic level with sodium hydroxide and then extracted with hexane. The extract was washed with saturated brine and dried over anhydrous sodium sulfate. By evaporating the solvent, 0.34 g of the title compound was obtained. The yield was 83%.
¹H-NMR δ (ppm); 1.43 (3H, d), 4.73 (1H, q), 5.30 (2H, br), 7.4 - 7.6 (4H, m)

### INDUSTRIAL APPLICABILITY

According to the present invention, the methoxyiminoacetamide derivative of general formula (I) which is useful as an agricultural horticultural fungicide can be produced with high efficiency through the novel intermediate acetal derivative of general formula (III). According to the present invention, it is possible to provide the methoxyiminoacetamide derivative of interest with a high yield of about 90%.

As described in an unexamined published Japanese patent application (*kokai*) No. 7-076564, the methoxyiminoacetamide derivative obtained by the present invention is an excellent agricultural horticultural fungicide having high controlling effect, for example, against diseases such as powdery mildew, brown rust, etc. of wheat and the like.

## Claims

1. A method for producing an N-methyl-methoxyiminoacetamide derivative represented by the following general formula (I) 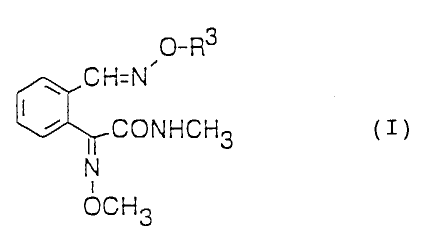 (wherein R³ represents a hydrogen atom or a group represented by a general formula -C(R⁴)(R⁵)-Ar (wherein each of R⁴ and R⁵ independently represents a hydrogen atom or a C₁-C₄ alkyl group, and Ar represents an optionally substituted aryl group or an optionally substituted heteroaryl group)), which comprises allowing an acetal derivative represented by the following general formula (III) 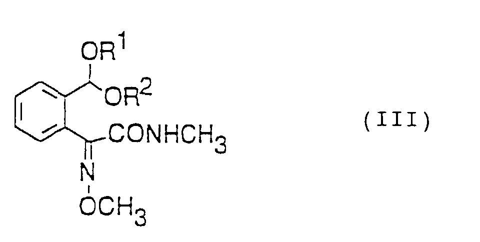 (wherein R¹ and R² independently represent a C₁-C₄ alkyl group, or R¹ and R² in combination represent a C₂-C₄ alkylene group) to react with an oxyamine derivative represented by the following general formula (II)
H₂NO-R³ (II)
(wherein R³ is as described above).

2. The method for producing an N-methyl-methoxyiminoacetamide derivative according to claim 1, wherein the acetal derivative represented by the general formula (III) is obtained by allowing a methoxyiminoacetic acid ester derivative represented by the following general formula (IV) 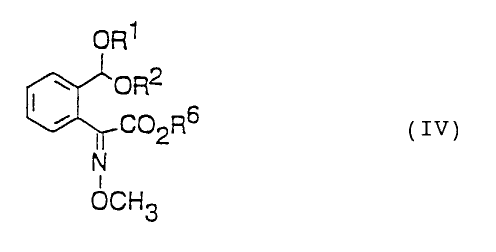 (wherein R¹ and R₂ are as defined in the general formula (III), and R⁶ represents a C₁-C₄ alkyl group) to react with methylamine.

3. The method for producing an N-methyl-methoxyiminoacetamide derivative according to claim 1, wherein the acetal derivative represented by the general formula (III) is obtained by allowing the methoxyiminoacetic acid ester derivative represented by the general formula (IV) to react with methylamine, said methoxyiminoacetic acid ester derivative being obtained by allowing a 2-(2-formylphenyl)-2-methoxyiminoacetic acid ester represented by the following general formula (V) 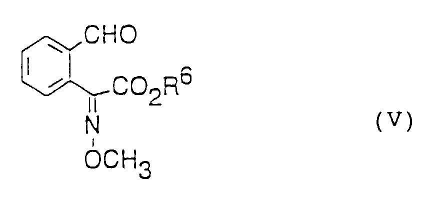 (wherein R⁶ is as defined in the general formula (IV)) to react with an alcohol or an orthoformic acid ester.

4. The method for producing an N-methyl-methoxyiminoacetamide derivative according to claim 1, wherein the acetal derivative represented by the general formula (III) is obtained by allowing the methoxyiminoacetic acid ester derivative represented by the general formula (IV) to react with methylamine, said methoxyiminoacetic acid ester derivative being obtained by allowing the 2-(2-formylphenyl)-2-methoxyiminoacetic acid ester represented by the general formula (V), which is obtained by treating a 2-(2-alkoxymethylphenyl)-2-methoxyiminoacetic acid ester represented by the following general formula (VI) 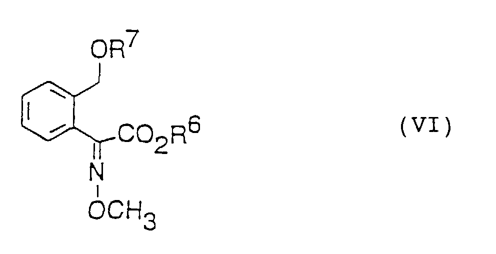 (wherein R⁷ represents a C₁-C₄ alkyl group and R⁶ is as defined in the general formula (IV)) with a halogenation agent, to react with an alcohol or an orthoformic acid ester.

5. An acetal derivative which is represented by the general formula (III).

6. The method for producing an N-methyl-methoxyiminoacetamide derivative according to claim 1, wherein R¹ and R² in the general formula (III) are methyl or ethyl, or R¹ and R² in combination represent ethylene.

7. The method for producing an N-methyl-methoxyiminoacetamide derivative according to claim 1, wherein R³ in the general formula (II) is a group represented by a general formula -C(R⁴)(R⁵)-Ar wherein R⁴ and R⁵ are independently a hydrogen atom or a methyl group and Ar is a phenyl group which may be substituted with a substituent selected from a C₁-C₄ alkyl group, a halogen atom, a haloalkoxy group and trifluoromethyl group.

8. The acetal derivative according to claim 5, wherein R¹ and R² in the general formula (III) are methyl or ethyl, or R¹ and R² in combination represent ethylene.

9. A method for producing a 2-(2-formylphenyl)-2-methoxyiminoacetic acid ester represented by the following general formula (V) 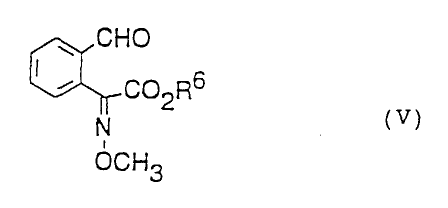 (wherein R⁶ is as defined in the foregoing), which comprises treating a 2-(2-alkoxymethylphenyl)-2-methoxyiminoacetic acid ester represented by the following general formula (VI) 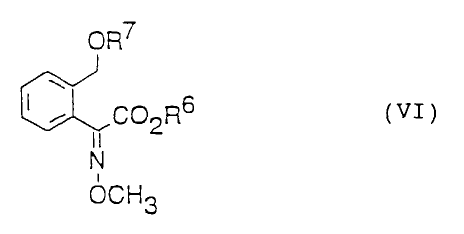 (wherein R⁶ and R⁷ independently represent a C₁-C₄ alkyl group) with a halogenation agent.

10. The method for producing a 2-(2-formylphenyl)-2-methoxyiminoacetic acid ester according to claim 9, wherein a base is allowed to exist when the treatment with a halogenation agent is carried out.
